# EUROPEAN PATENT APPLICATION

(11) **EP 4 198 121 A1**
(43) Date of publication of application: **21.06.2023**
(21) Application number: 21215254.0
(22) Date of filing: 16.12.2021
(51) Int. Cl.: C12N 5/0793, C12N 5/079, C12N 5/078, C12N 5/071

(54) **A METHOD FOR OBTAINING A NEUROMUSCULAR ORGANOID AND USE THEREOF**

(71) Applicant: Universiteit Antwerpen, 2000 Antwerpen (BE)
(72) Inventor: Van Lent, Jonas, 2000 Antwerp (BE); Timmerman, Vincent, 2000 Antwerp (BE)
(74) Representative: Arnold & Siedsma

(57) **Abstract**

The invention is situated in the field of cell culturing and disease modeling. More specifically, the invention relates to a method for obtaining a neuromuscular 3D organoid demonstrating myelination that is derived from stem cells. The invention further relates to a neuromuscular organoid obtained using the method of the invention which can be used to study (peripheral) neuropathic diseases, in particular Charcot-Marie-Tooth disease characterized by myelination and/or axonal defects.

## Description

### FIELD OF THE INVENTION

The invention is situated in the field of cell culturing and disease modeling. More specifically, the invention relates to a method for obtaining a neuromuscular 3D organoid demonstrating myelination that is derived from stem cells. The invention further relates to a neuromuscular organoid obtained using the method of the invention which can be used to study (peripheral) neuropathic diseases, in particular Charcot-Marie-Tooth disease characterized by myelination and/or axonal defects.

### BACKGROUND OF THE INVENTION

Charcot-Marie-Tooth (CMT) disease is the most common inherited peripheral neuropathy affecting approximately 2.6 million people worldwide. Being a clinically and genetic heterogeneous hereditary neuropathy, CMT is mainly classified in demyelinating (CMT1) and axonal (CMT2) forms.

Induced pluripotent stem cells (iPSCs) form a powerful tool to study CMT, however, the complex process of myelination cannot be mimicked by such monocultures, as myelination involves the engulfment of motor and sensory neurons by Schwann cells. The formation of such peripheral nervous system model would therefore require the presence of multiple cell types in the same dish. Attempts to study demyelinating diseases in vitro have so far relied on co-cultures between neurons and myelinating Schwann cells from rats. This model has its obvious shortcomings as it cannot be derived from human cells. Moreover, it does not include muscle cells, an essential component, to which neurons connect through the formation of functional neuromuscular junctions (NMJs). In summary, current approaches to study disease modeling encounter the problem that number of biological phenomena that are specific to complex human systems are not amenable to being reproduced in animal models or even 2D cultures.

The present invention is intended to solve the above problems and an object of the present invention is to obtain a human model to study the peripheral nervous system, including myelinating Schwann cells and other relevant cell types. The object is solved by the subject matter of the claims. In particular, the object underlying the present invention is solved according to a method of obtaining a neuromuscular 3D organoid comprising the steps of: a) obtaining pluripotent stem cells (PSC); b) culturing said PSCs in a cell culture substrate; c) resuspending the cells of step b) in an organoid differentiation substrate; d) seeding the resuspended cells of step c) as a non-adherent culture; e) contacting the non-adherent culture of step d) with said organoid differentiation substrate until a3D organoid is formed; f) transferring said formed 3D organoid to a surface comprising a basement membrane matrix; wherein during step d) to step f) said non-adherent culture is maintained on a shaker. In particular, said organoid differentiation substrate is capable to form neuronal cells that can be myelinated in a neuromuscular structure.

Organoids are three-dimensional cell cultures from embryonic stem cells or induced pluripotent stem cells with the potential to self-organize into in vivo-like organ complexes and the ability to recapitulate essential organ function. Since organoids are derived from human stem cells, they provide the genetic and physiological similarity required for employment in biomedical research such as modeling of human development and disease, drug screening, regenerative medicine. Organoids have enormous advantages compared to 2D cultures and animal models, which make them a practical platform for different experiments, modeling diseases, and high-throughput screening. For example, organoids are easy to manipulate, have a high heterogeneity, can model cellular/mechanical communications and human physiology, and can be generated from the organ of interest or diseases patient tissue.

The invention further provides a neuromuscular organoid obtained using the method of the invention, wherein said organoid is able to show myelination. The neuromuscular organoid can be used to study (peripheral) neuropathic diseases, in particular Charcot-Marie-Tooth disease that is characterized by myelination and/or axonal defects.

The inventors have surprisingly found that a neuromuscular organoid obtained by the method of the present invention contains neurons, muscle cells, Schwann cells, endothelial cells and glial cells. Furthermore, the organoid model contains myelinated neurons and forms neuromuscular junctions (NMJs), capturing key features of the peripheral nervous system. The assessment of CMT1A organoids reveals key features of the CMT1A disease pathology. This self-organizing organoid model is therefore able to capture the physiological complexity of the peripheral nervous system and forms an excellent model to study demyelinating diseases.

### SUMMARY OF THE INVENTION

In a first aspect, the present invention relates to a method of obtaining a neuromuscular 3D organoid, the method comprising:
a) Obtaining pluripotent stem cells (PSC);
b) Culturing said PSCs in a cell culture substrate;
c) Resuspending the cells of step b) in an organoid differentiation substrate;
d) Seeding the resuspended cells of step c) as a non-adherent culture;
e) Contacting the non-adherent culture of step d) with said organoid differentiation substrate until a neuromuscular 3D organoid is formed;
f) transferring said formed neuromuscular 3D organoid to a surface comprising a basement membrane matrix;
   wherein during step d) to step f) said non-adherent culture is maintained on a shaker.

In particular, said organoid differentiation substrate is capable to form neuronal cells that can be myelinated in a neuromuscular structure.

In a specific embodiment of the method of the present invention, said organoid differentiation substrate comprises one or more reagents selected from the list comprising STEMdiff APEL2 medium, Protein Free Hybridoma Medium, NB medium, a GSK-3 Inhibitor, basic fibroblast growth factor (bFGF2), Forskolin, ROCK Inhibitor, B27 supplement, N2 supplement, L-glutamine, neurobasal medium, BSA fraction V, 2-mercaptoethanol, in particular STEMdiff APEL2 medium and Protein Free Hybridoma Medium.

In another specific embodiment of the method of the present invention, the organoid differentiation substrate comprises one or more reagents selected from the list comprising a GSK-3 Inhibitor, basic fibroblast growth factor (bFGF2), Forskolin.

In yet a further embodiment of the method of the present invention, the seeding of step d) is performed on a surface pre-treated with an anti-adherence rinsing solution.

In a particular embodiment of the method of the present invention, said non-adherent culture is centrifuged after seeding of step d), in particular for 3 min at 100G.

In a particular embodiment, the said shaker is an orbital shaker, in particular an orbital shaker rotating at a speed ranging between 25 and 200 rpm, preferably 70 rpm.

In another embodiment, the method of the present invention further comprises dissociating said cultured PSCs into single cells prior to the resuspending of step c), in particular by a cell detachment solution, in particular accutase.

In yet another embodiment of the method of the present invention, the seeding of step d) is performed at a density of at least 300 000 cells/mL resuspended cells.

In a following embodiment of the method of the present invention, the contacting of said organoid differentiation substrate on day 1 of culturing is at a three-fold higher concentration of supplements compared to the organoid medium.

In a specific embodiment of the method of the present invention, said non-adherent culture of step e) is re-plated after 7 days of culturing to a low-binding surface, in particular a ultra-low attachment plate.

In yet a further embodiment of the method of the present invention, the re-plating of the non-adherent culture is at a density of 22 spheres per well.

In a particular embodiment of the method of the present invention, after 9 days of culturing, said non-adherent culture is contacted with a cell grow medium, in particular DMEM, more in particular DMEM with high glucose supplemented with horse serum in particular DMEM with high glucose supplemented with 2% horse serum.

In another embodiment of the method of the present invention, said basement membrane matrix is Matrigel or Geltrex.

In yet another embodiment of the method of the present invention, said PSCs are obtained from a human subject having a neuromuscular disease, in particular a peripheral neuropathy disease, more in particular Charcot-Marie-Tooth disease.

In a further aspect, the present invention provides a neuromuscular organoid obtained using the method of the present invention.

In yet another aspect, the present invention provides a neuromuscular organoid for use to study neuropathic diseases, in particular CMT1A (myelination), CMT1B, CMT2A (aconopathy), CMT3-6, CMTX.

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.

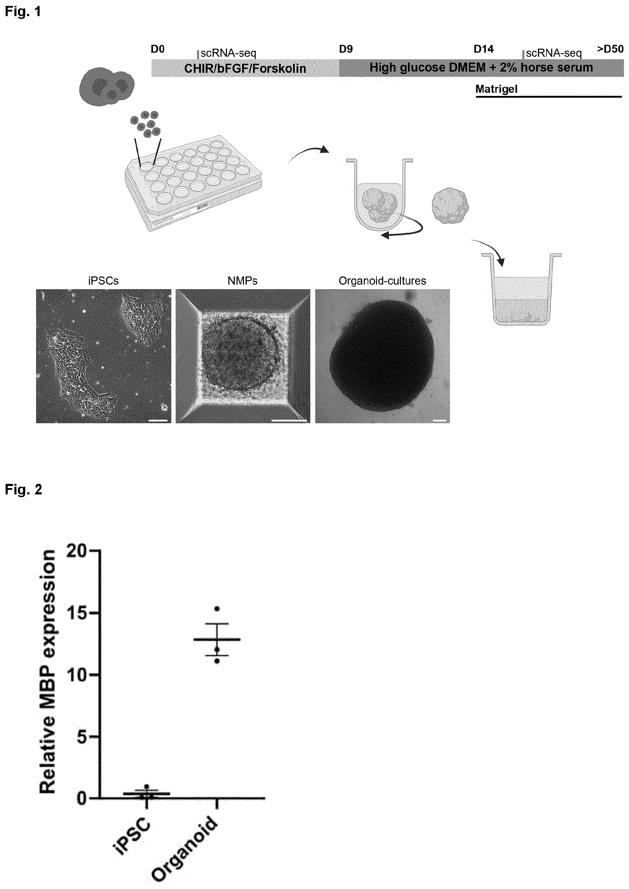

**Fig. 1****: Generation of iPSC-derived organoid-cultures.** Graphic scheme of procedures for the generation of iPSC-derived organoid-cultures. The iPSCs were dissociated in single cells and aggregated in microwells. Cells were transferred into an ultra-low attachment plate on a shaker, and afterwards grown as 3D-cultures in Matrigel. On top: the compounds used to obtain 3D-cultures. Below: phase-contract images of the different stages. *Abbreviations: iPSC induced pluripotent stem cell, CHIR CHIR99021, bFGF basic fibroblast growth factor and NMP neuromesodermal progenitor. Figure created with BioRender.*
**Fig. 2****: Characterization of organoid-cultures.** RT-qPCR to investigate the relative expression of MBP in iPSC colonies at Day 0, and at mature organoid stages *in vitro* (*n*=3; data pooled from two independent differentiations; mean ± SEM). Statistical significance of RT-qPCR results was calculated using the Mann-Whitney U test (* *P*<0.05, ** *P*<0.01, *** *P*<0.001, **** *P*<0.0001).

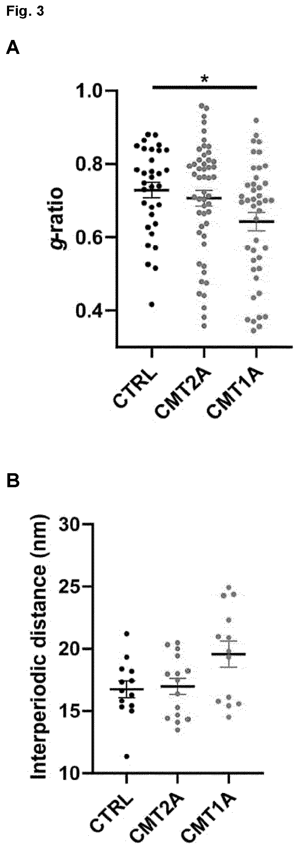

**Fig. 3****: CMT1A-derived organoids cause myelin alterations.** (**A**) Quantification of *g*-ratio at Days 18-25 of myelination. Each dot represents individual myelinated neurons (*n*=32-51; data pooled from two independent differentiations; mean ± SEM). (**B**) Quantification of interperiodic line distance at Days 18-25 of myelination. Each dot represents the average value of measured interperiodic line distances per myelinated neuron (*n*=13-15; data pooled from two independent differentiations;
mean ± SEM). Statistical significance to evaluate *g*-ratio was performed using one-way ANOVA followed by Dunnett's multiple comparisons test, while Kruskal-Wallis test with Dunn's multiple comparison was used to perform statistics on the interperiodic line distance (* *P*<0.05, ** *P*<0.01, *** *P*<0.001, **** *P*<0.0001).

### DETAILED DESCRIPTION OF THE INVENTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

As used in the specification and the appended claims, the singular forms "a", "an", and "the" include plural referents unless the context clearly dictates otherwise. By way of example, "a compound" means one compound or more than one compound.

The terms "comprising", "comprises" and "comprised of" as used herein are synonymous with "including", "includes" or "containing", "contains", and are inclusive or open-ended and do not exclude additional, non-recited members, elements, or method steps. The terms also encompass "consisting of" and "consisting essentially of".

The term "about" or "approximately" as used herein when referring to a measurable value such as a parameter, an amount, a temporal duration, and the like, is meant to encompass variations of +/-10% or less, preferably +/-5% or less, more preferably +/- 1 % or less, and still more preferably +/-0.1 % or less of and from the specified value, insofar such variations are appropriate to perform in the disclosed invention. It is to be understood that the value to which the modifier "about" or "approximately" refers is itself also specifically, and preferably, disclosed.

First, some definitions are disclosed with non-limiting embodiments of a neuromuscular organoid able to show a myelination process. Secondly, non-limiting embodiments of a method of obtaining said neuromuscular organoid are disclosed thereafter.

In a first aspect, the present invention relates to a method of obtaining a neuromuscular 3D organoid, the method comprising: a) obtaining pluripotent stem cells (PSCs); b) culturing said PSCs in cell culture substrate; c) resuspending the cells of step b) in an organoid differentiation substrate; d) seeding the resuspended cells of step c) as a non-adherent culture; e) contacting the non-adherent culture of step d) with said organoid differentiation substrate until a neuromuscular 3D organoid is formed; f) transferring said formed neuromuscular 3D organoid to a surface comprising a basement membrane matrix; wherein during step d) to step f) said non-adherent culture is maintained on a shaker.

In a particular embodiment, said organoid differentiation substrate results in the formation of neuronal cells which can be myelinated in a neuromuscular structure.

Accordingly, step c) of the method is preferably performed until at least part of said pluripotent stem cells is differentiated into neuronal cells.

In a further aspect, the present invention provides a neuromuscular organoid obtained using the method of the present invention.

As used herein, the term "organoid" refers to a miniature organ made by culturing or recombining isolated cells. The term is to be interpreted broadly to include a three-dimensional (3D) multicellular *in vitro* tissue construct that substantially mimics its corresponding *in vivo* tissue or organ. Therefore, an organoid may contain multiple cell types and mimics physiological conditions/ structures observed *in vivo* in a tissue or an organ of a subject. This is in contrast with two-dimensional (2D) cultures that typically contain fewer cell types and do not mimic physiological conditions/ structures observed in vivo in a tissue or an organ. The organoid may be used to study aspects of that tissue or organ in a tissue culture dish. The term *"in vitro* " refers to an artificial environment and to processes or reactions that occur within an artificial environment, or elsewhere outside a living organism. *In vitro* environments may include, but are not limited to, test tubes, cell cultures, well-plates, bioreactors etc.

The term "neuromuscular" as used herein refers to both nerves and muscles as in the neuromuscular junction (NMJ) (the meeting place of a nerve and a muscle fiber), and neuromuscular transmission (the transfer of "information" from the nerve to the muscle). The NMJ is a highly organized chemical synapse formed between motor neurons (MNs) and muscles and it contains an additional important cell type, the Schwann cells able to myelinate neuronal axons.

As described herein, the organoid is self-organized in 3D and comprises all different cell types required for the formation of functional NMJs, to appropriately model the mechanisms of neuromuscular diseases, particularly those in which the early stages of NMJ formation are impaired. Furthermore, as described in the examples, the neuromuscular organoids have NMJs as demonstrated a fluorescent conjugate for sensitive detection of neuromuscular junctions e.g. by α-Bungarotoxin-FITC.

In a particular embodiment, the neuromuscular organoid of the present invention comprises or consists of Schwann cells, motor neurons, sensory neurons, muscle cells, endothelial cells, and glial cells.

In some embodiments, the neuromuscular organoid further comprises at least one functional neuromuscular junction and/or at least one functional myelinating neuron.

The term "pluripotent stem cells" ("PSCs") as used herein refer to cells that have the capacity to self-renew by dividing and to develop into the three primary germ cell layers of the early embryo (endoderm, mesoderm and ectoderm) and therefore into all cells of the adult body, but not extra-embryonic tissues such as the placenta.

In some embodiments, said pluripotent stem cells (PSCs) of the present invention may be embryonic stem cells or induced pluripotent stem cells (iPSCs).

The term "embryonic stem cells" ("ESCs") as used herein refer to stem cells derived from the inner cell mass of preimplantation embryos during *in vitro* fertilization as well as embryonic stem cells made by somatic cell nuclear transfer and embryonic stem cells from unfertilized eggs. The stem cells may be derived from sources which include but are not limited to embryonic or fetal tissue, post-fetal tissue, adult tissue and differentiated tissue. ES cells have also the potential to self-renew and differentiate into mature cells such as but not limited to neural cells, muscle cells, adipocyte cells, hepatocytes, organ cells, blood cells, bone cells.

The term "induced pluripotent stem cells" ("iPSCs") as used herein refer to stem cells induced from a somatic cell, e.g., a differentiated somatic cell, and that has a higher potency than said somatic cell. Like ESCs, iPSCs are capable of self- renewal and differentiation into mature cells. iPSCs of the present invention may be obtained by methods described in the state of art (US8058065), for example by somatic cell reprogramming following the exogenous expression of specific transcription factors Oct-3/4, KLF4, SOX2, and c-Myc (commonly known as Yamanaka factors). Within the context of the present invention, the term "somatic cell" refers to essentially all the cells of the body except for the germ line; the germ line being the cells in the sexual organs that produce sperm and eggs. Examples of somatic cells are cells of internal organs, skin, bones, blood such as peripheral blood mononuclear cells (PBMCs); or connective tissue such as fibroblasts. Fibroblasts and PBMCs are most commonly used to produce human iPSCs.

Thus, within the context of the present invention, the term PSCs will be used to refer to actual PSCs as well as iPSCs or ESCs. Furthermore, since somatic cells can be reprogrammed into iPSCs, the method of the present invention also encapsulated the step of obtaining somatic cells from a patient or cells line and reprogram the cells to iPSCs to be used to for culturing in a cell culture substrate. The term "differentiation" as used herein refers to a process during which the default cell type (genotype and/or phenotype) changes to a non-default cell type (genotype and/or phenotype). During cell differentiation, the changes may include cell shape, cell size, membrane potential, metabolic activities, and responsiveness which are induced by modifications in gene expression. Thus "inducing differentiation" refers to inducing the cell to divide into progeny cells with characteristics that are different from the stem cell, such as genotype (i.e. change in gene expression) and/or phenotype (i.e. change in expression of a protein).

In the context of the present invention, iPSCs are differentiated to neuromesodermal progenitors (NMPs) by resuspending said iPSCs in an organoid differentiation substrate capable of neuromuscular signaling. The term "neuromuscular signaling" as used herein refers to a process wherein iPSCs are stimulated to acquire a neural fate such as neuroectodermal and mesodermal cell derivatives rather than give rise to other structures such endodermal structures such as for example lung cells. In particular, within the organoid, a neuromuscular structure is formed comprising Schwann cells that are able to myelinate neuronal cells. NMPs are the source of new axial structures during embryonic rostrocaudal axis elongation and are marked by the simultaneous expression of the transcription factors T(Brachyury) (T(Bra)) and Sox2 (see example part).

In some embodiments, iPSCs can be derived from any healthy or diseased individual. For example, as described in the example part, human iPSCs lines were derived from a healthy individual as well as cell lines derived from patients with Charcot-Marie-Tooth disease e.g. CS67iCMT for CMT1A or c.281G>A for CMT2A.

In yet another embodiment of the method of the present invention, said PSCs are obtained from a human subject having a neuromuscular disease, in particular a peripheral neuropathy disease, more in particular Charcot-Marie-Tooth disease.

A used herein, the term "peripheral neuropathy" refers to the many conditions that involve damage to the peripheral nervous system, the vast communication network that sends signals between the central nervous system (the brain and spinal cord) and all other parts of the body.

In one embodiment, the neuromuscular disease is a peripheral neuropathy disease selected from the list comprising Carpal tunnel syndrome, Ulnar neuropathy, Radial neuropathy, Peroneal neuropathy, Brachial plexus neuropathy or hereditary neuralgic amyotrophy (HNA), Meralgia paraesthetica, Facial nerve (Bell's) palsy, Sixth-nerve palsy, Fourth-nerve palsy, Third-nerve palsy, Mononeuritis multiplex, Multifocal motor neuropathy, Guillain-Barre syndrome, Chronic polyneuropathy, Diabetic polyneuropathy, Idiopathic polyneuropathy, Charcot-Marie-Tooth disease (CMT), hereditary neuropathy with liability to pressure palsies (HNPP), chronic inflammatory demyelinating polyneuropathy (CIDP), Amyotrophic lateral sclerosis (Lou Gehrig's Disease), Lambert-Eaton syndrome, Muscular dystrophies, Myasthenia gravis (MG), Myopathies, distal myopathies, sensory and autonomic neuropathies (HSAN, also known as HSN), hereditary motor and sensory neuropathies (HMSN), distal hereditary motor neuropathies (HMN).

In one embodiment, said PSCs are obtained from a human subject having Charcot-Marie-Tooth disease.

In another embodiment, said PSCs are obtained from a human subject having HMSN.

In another embodiment, said PSCs are obtained from a human subject having HSN.

In another embodiment, said PSCs are obtained from a human subject having HMN.

In another embodiment, said PSCs are obtained from a human subject having HNPP.

As used herein, the term "Charcot-Marie-Tooth disease" ("CMT") refers to a hereditary motor and sensory neuropathy of the peripheral nervous system characterized by progressive loss of muscle tissue and touch sensation across various parts of the body. Being a clinically and genetic heterogeneous hereditary neuropathy, CMT is mainly classified in demyelinating (CMT1) and axonal (CMT2) forms. CMTX is a form of CMT that is principally inherited in an X-linked pattern. CMT3 and CMT4 are rare types of CMT that disrupts the myelin sheath; CMT3 is characterized by severe, and early-onset (childhood).

In yet another aspect, the present invention provides a neuromuscular organoid for use to study neuropathic diseases, in particular CMT1A (myelination), CMT1B, CMT2A (axonopathy), CMT3, CMT4 or, CMTX, alternatively HMSN, HMN or HSN.

In some other embodiments, the organoid of the present invention is used to study human development and disease.

In another embodiment, the organoid of the present invention is used as a platform for screening agents that affect the cells within the organoid e.g. drug screening platform.

In another embodiment, the organoid of the present invention may provide a platform for testing molecules or agents that modulate e.g. promote or inhibit motor neuron survival.

In yet another embodiment, the organoid of the present invention is used in regenerative medicine.

While the method of the present invention is particularly suitable for obtaining neuromuscular organoids, it may also be suitably to obtain other types of organoids such as but not limited to thyroid organoid, thymic organoid, testicular organoid, prostate organoid, hepatic organoid, pancreatic organoid, intestine organoid, stomach organoid, epithelial organoid, lung organoid, kidney organoid, gastruloid (embryonic organoid), blastoid (blastocyst-like organoid), cardiac organoid, retinal organoid, glioblastoma organoid, spinal organoid, or any organoid mimicking other tissue types. The skilled artisan is aware that ESCs or iPSCs derived from a specific organ type or cell line, optionally in combination with suitable organoid differentiation substrates will lead to generation of other types of organoid, and this, the method of the present invention is not limited to obtaining a neuromuscular organoid.

Now, the method of obtaining a neuromuscular organoid will be disclosed. A general concept of a method for preparing a (patient) iPSC-derived neuromuscular organoid model according to the present invention is illustrated in **Fig. 1****.** Overall, the process of organoid formation involves three crucial phases. First phase, PSCs are obtained (step a) and cultures in a cell culture substrate (step b). Second phase, the cultured PSCs are resuspended in an organoid differentiation substrate, which is preferably capable to form neuronal cells that can be myelinated in a neuromuscular structure (step c), seeding them as a non-adherent culture (step d) and contacting the culture with said differentiation substrate until a neuromuscular organoid is formed (step e). Third phase, the formed neuromuscular 3D organoid is transferred to a surface comprising a basement membrane matrix for future maturation (step f).

In the first phase of the method of the present invention, PSCs or iPSCs are obtained and cultured in a cell culture substrate. As used herein, the term "culturing" refers to the process wherein tissue-derived isolated cells are maintained or grown outside their natural environment under carefully controlled conditions. These conditions vary for each cell type, but generally consist of a suitable container with a substrate or medium that supplies the essential nutrients (amino acids, carbohydrates, vitamins, minerals), growth factors, hormones, and gases (CO₂, O₂), and regulates the physio-chemical environment (pH buffer, osmotic pressure, temperature). Culturing iPSC lines are known by those skilled in the art, for example maintaining said cultured iPSC lines on Matrigel coated plates in commercially available culture media such as Essential 8 Flex medium and supplement.

In some embodiments, the cell culture substrate of the present invention can be Essential 8 Flex medium, optionally with a supplement, but is not limited thereto.

In another embodiment, a supplement can be used to enhance cell survival upon reviving of said cultured PSCs such as for example RevitaCell supplement.

In another embodiment, the method of the present invention further comprises dissociating said cultured PSCs into single cells prior to resuspending, in particular by a cell detachment solution, in particular accutase. The cell detachment solution contains proteolytic and collagenolytic enzymes for the usage of detachment of cells from standard tissue culture plates or T-flask.

In one embodiment, other cell detachment solutions can be used such as, but not limited to, Trypsin/EDTA, TrypLE selection agent, IV type collagenase and neutral protease or other mechanical methods known in the state of art, for example using mechanical tools such as a cell scraper.

In one embodiment, cultured PSCs were detached from said culture plate when at least 40%, 50%, 60%, 70%, 80%, 90, 100%, in particular at least 80% confluency was reached. As used herein, the term "confluency" refers to the percentage of the surface of a culture plate that is covered by adherent cells.

In a second phase of the method of the present invention, the cultured PSCs are resuspended in an organoid differentiation substrate capable of neuromuscular signaling, seeding them as a non-adherent culture and contacting the culture with said differentiation substrate until a neuromuscular organoid is formed.

As used herein, the term "organoid differentiation substrate" refers to a substrate that comprises one or more morphogens and/or signaling inhibitors to activate or inhibit key signaling pathways that regulate developmental patterning in order to establish a correct regional identity during stem cell differentiation.

In a specific embodiment of the method of the present invention, said organoid differentiation substrate comprises one or more reagents selected from the list comprising STEMdiff APEL2 medium, Protein Free Hybridoma Medium, NB medium, a GSK-3 Inhibitor, basic fibroblast growth factor (bFGF2), Forskolin, ROCK Inhibitor, B27 supplement, N2 supplement, L-glutamine, neurobasal medium, BSA fraction V, 2-mercaptoethanol or any combination thereof.

In the context of the present invention, the organoid differentiation substrate comprises a basic organoid medium, from hereon termed "organoid medium" such as for example STEMdiff APEL2 medium, Protein Free Hybridoma Medium, NB medium, or any other suitable medium in combination with supplements, from here on termed "supplements", for example a GSK-3 Inhibitor, basic fibroblast growth factor (bFGF2), Forskolin, ROCK Inhibitor, B27 supplement, N2 supplement, L-glutamine, neurobasal medium, BSA fraction V, 2-mercaptoethanol. The term "reagents" can be used interchangeably for both an organoid medium or a supplement of the organoid differentiation substrate.

In another specific embodiment of the method of the present invention, said organoid differentiation substrate comprises one or more reagents selected from the list comprising a GSK-3 Inhibitor, basic fibroblast growth factor (FGF2), Forskolin.

In a particular embodiment, the organoid differentiation substrate of the present invention comprises STEMdiff APEL2 medium and Protein Free Hybridoma Medium supplemented with a GSK-3 Inhibitor, basic fibroblast growth factor (bFGF2), Forskolin, and ROCK Inhibitor.

The method of the present invention is not limited to the use of STEMdiff APEL2 medium since this medium is known by the skilled person as a fully defined, serum-free and animal component-free medium for the differentiation of human embryonic stem cells and induced pluripotent stem cells. Thus, it can easily be replaced by another supporting differentiating medium with or without a Protein Free Hybridoma Medium.

As used herein, the term "GSK-3" refers to a glycogen synthase kinase 3 (GSK-3) which is a serine/threonine protein kinase that phosphorylate either threonine or serine, and this phosphorylation controls a variety of biological activities, such as glycogen metabolism, cell signaling, cellular transport, and others. A GSK3 inhibitor is an agent that activates Wnt signaling pathways and plays a role in the transformation from one cell type to another. The term broadly refers to any factor that effects, increases, induces, initiates, or stimulates release of a Wnt protein; a factor that effects or produces biochemical signaling within a Wnt signaling pathway; and a factor that increases, induces, initiates, or stimulates signaling within a Wnt signaling pathway.

In some embodiments, GSK-3 inhibitors can be different chemotypes that have variable mechanisms of action; they may be cations, from natural sources, synthetic ATP and non-ATP competitive inhibitors and substrate-competitive inhibitors. In some embodiments, synthetic ATP GSK-3 inhibitors can be selected from the groups comprising marine organism-derived inhibitors, aminopyrimidines, arylindolemaleimide, thiazoles, paullones or aloisines. In some embodiments, synthetic non-ATP GSK-3 inhibitors can be selected from the groups comprising marine organism-derived inhibitors, thiazolidinediones, halomethylketones, peptides, or small molecule inhibitors.

Examples of suitable GSK-3 inhibitors maybe one or more selected from, but not limited to, beryllium, copper, lithium, mercury or tungsten 6-Bromoindirubin-3'-oxime (BIO), Dibromocantharelline, Hymenialdesine, Indirubin, Meridianin, CHIR99021, CT98014, CT98023, CT99021, TWS119, 3-(2,4-Dichlorophenyl)-4-(1 - methyl-1 H-indol-3-yl)-1 H-pyrrole-2,5-dione (SB 216763), SB-41528, N-(4-Methoxybenzyl)- N'-(5-nitro-1 ,3-thiazol-2-yl)urea (AR-AO 14418), AZD-1080, Alsterpaullone, Cazpaullone, Kenpaullone or aloisines, Manzamine A, Palinurine, Tricantine, 4-Benzyl-2-methyl- 1 ,2,4-thiadiazolidine-3,5-dione (TDZD-8), NP00111, NP031115, Tideglusib, HMK-32, L803-mts, L807-mts, COB-187 or COB-152.

In a particular embodiment of the present invention, the GSK-3 Inhibitor is CHIR99021.

Another embodiment of the invention regards a method, wherein the concentration of the GSK-3 Inhibitor is in the range of about 0.01 to about 10 µM, in particular in the range of about 0.05 µM to about 5 µM, preferably in the range of about 0.1 µM to about 1 µM, more preferably about 0.5 µM.

In some embodiments, the concentration of the Protein Free Hybridoma Medium is in the range of about 0.5% to about 20%, in particular in the range of about 1% to about 10%, preferably in the range about 4% to about 7 %, more preferably about 5.32%.

In some embodiments, the concentration of bFGF2 is in the range of about 0.1 ng/mL to about 40 ng/mL, in particular in the range of about 1 ng/mL to about 20 ng/mL, preferably in the range of about 5 ng/mL to about 15 ng/mL, more preferably about 10 ng/mL.

In some embodiments, the concentration of the Forskolin is in the range of about 0.1 µM to about 80 µM, in particular in the range of about 1 µM to about 40 µM, preferably in the range of about 10 µM to about 30 µM, more preferably about 20 µM.

In some embodiments, the concentration of the ROCK inhibitor is in the range of about 0.1 µM to about 40 µM, in particular in the range of about 1 µM to about 20 µM, preferably in the range of about 5 µM to about 15 µM, more preferably about 10 µM.

In some embodiments of the present invention, the ROCK inhibitor may include, but is not limited to, Y-27632 ((1R,4r)-4-((R)-1-aminoethyl)-N-(pyridin-4-yl)cyclohexanecarboxamide). The ROCK inhibitor plays a role in inhibiting apoptosis and cell differentiation, and the size and shape of an organoid may vary depending on presence or absence of the ROCK inhibitor.

In specific embodiment, the organoid differentiation substrate of the present invention comprises STEMdiff APEL2 medium and 5.32% Protein Free Hybridoma Medium supplemented with 0.5 µM GSK-3 Inhibitor, 10 ng/mL basic fibroblast growth factor (bFGF2), 20 µM Forskolin, and 10 µM ROCK Inhibitor.

In another particular embodiments, the organoid culture step of the present invention may also be carried out using an organoid differentiation substrate comprising B27 supplement, N2 supplement, human epidermal growth factor (hEGF), and human fibroblast growth factor (hFGF).

In some embodiments, the organoid differentiation substrate of the present invention may comprise Matrigel, collagen, alginate, agarose, gelatin, fibrin, hyaluronic acid, chitosan or a mixture of two or more thereof.

In some embodiments, the cultured PSCs are resuspended in an organoid differentiation substrate. Specifically, in the resuspending step, the organoid may be dispensed into each well of the well plate to which the organoid differentiation substrate has been added, and then cultured until the cells are caused to form a 3-dimensional shape.

In yet another embodiment of the method of the present invention, the seeding of step d) is performed at a density of at least 300 000 cells/mL resuspended cells.

In some embodiments, the stem cells are seeded at a density of at least about 50,000 cells/mL, at least about 100,000 cells/mL, at least about 150,000 cells/mL, at least about 200,000 cells/mL, at least about 250,000 cells/mL, at least about 300,000 cells/mL, at least about 350,000 cells/mL or at least about 400,000 cells/mL, preferably at least about 300 000 cells/mL in a differentiation substrate. In a case where the density of cells/ml is below the lower range, a problem may arise that the cells do not grow properly due to insufficient cell number per drop-in. In a case where the density of cells/ml is above the upper range, the number of cells per drop-in is excessive, so that the cells are detached from the cell culture substrate or have difficulty in forming a 3-dimensional structure. A seeding density of at least about 300,000 cells/mL may advantageously result in better derivation of mature cell types as compared to a lower seeding density.

In yet a further embodiment of the method of the present invention, the seeding of step d) is performed on a surface pre-treated with an anti-adherence rinsing solution. Within the context of the present invention, the term "anti-adherence rinsing solution" is meant to be a surfactant solution for pre-treating cultureware to reduce surface tension and prevent cell adhesion. Any commercially available anti-adherence rinsing solution known in the state of art can be suitable, for example a solution formerly known as Aggrewell Rinsing Solution.

In some embodiments, the differentiated cell culture is an adherent culture, a non-adherent culture, or a free-floating culture. As used herein, the term "free-floating culture" refers to cells that are not attached to a surface and are randomly floating in a suspension culture. In contrast, as used herein, the term "non-adherent culture" refers to cells in a suspending culture that are not attached to a surface but can be free-floating or can be settled down in well plate after e.g., centrifugation.

In some embodiments of the present invention, the non-adherent culture is centrifuged at about and between 1 and 500 G, about and between 20 and 400 G, about and between 40 and 300 G, about and between 50 and 200 G, preferably at about and between 60 and 150 G, more preferably at about and between 80 and 130 G, in particular at about 100 G as exemplified in the examples.

In another embodiment of the present invention, the non-adherent culture is centrifuged for about 0.5 minute, about 1 minute, about 2 minutes, about 3 minutes, about 4 minutes, about 5 minutes, about 6 minutes, about 7 minutes, about 8 minutes, about 9 minutes, about 10 minutes, about 20 minutes, about 30 minutes, abouts 60 minutes, in particular for about 2 minutes as exemplified in the examples.

In a particular embodiment of the method of the present invention, said non-adherent culture is centrifuged after seeding of step d), in particular for 3 min at 100G.

As used herein and unless otherwise specified, the term "centrifugation" is to be understood as rotating a cell plate, for example an ultra-low attachment plate, around its axis to precipitate cells, such that they are brought together and can thus more easily connect and grow. In a case where the non-adherent culture is not precipitated by centrifugation, a problem may be that cells do not easily connect and form organoids.

In some embodiments, from the seeding step onwards, the non-adherent culture is maintained on a shaker, in particular an orbital shaker. An advantage of using an orbital shaker is that during this period cells continue to mature while being kept in a non-adherent state with respect to the well surface and thus remain 3D.

In a particular embodiment, the orbital shaker rotates at a speed ranging about and between 25 and 500 rpm, about and between 30 and 400 rpm, about and between 35 and 300 rpm, about and between 40 and 200 rpm, about and between 45 and 100 rpm, about and between 50 and 80 rpm preferably 70 rpm.

In a particular embodiment, the orbital shaker rotates at a speed ranging about 20 rpm, 25 rpm, 30 rpm, 35 rpm, 40 rpm, 45 rpm, 50 rpm, 55 rpm, 60 rpm, 65 rpm, 70 rpm, 75 rpm, 80 rpm, 85 rpm, 90 rpm, 100 rpm.

In addition, in the present invention, the 3-dimensional shape of the organoid is preferably formed in a spherical shape. However, the present invention is not limited thereto.

In some embodiments, the PSCs are resuspended on day 0, wherein day 0 is defined as the day on which the PSCs are seeded as a non-adherent culture and contacted with the differentiation substrate in a cell plate for culturing.

In some embodiments, the organoid differentiation substrate capable of forming a 3D organoid is contacted to the non-adherent culture on day 1, day 2, day 3, day 4, day 5, day 6, day 7, day 8, day 9, day 10, day 11, day 12, day 13, day 14, day 15, day 16, day 17, day 18, day 19, day 20, day 21, day 22, day 23, day 24, day 25, day 26, day 27, day 28, day 29, day 30 of culture or any combination thereof.

In one embodiment, the organoid differentiation substrate capable of forming a 3D organoid is contacted to said non-adherent culture from day 1 to day 10 of culture.

In a preferred embodiment, the organoid differentiation substrate capable of forming a 3D organoid is contacted to said non-adherent culture from day 1 to day 7 of culture.

The term "contacting" as used herein in the context of contacting a cell or organoid with an agent or reagent e.g. a compound refers to placing the compound in a location that will allow it to touch the cell or organoid. Contacting may be accomplished using any suitable method. For example, contacting may be done by adding the compound to a tube containing the cell or organoid. Contacting may also be accomplished by adding the compound to a culture of the cell or organoid.

In some embodiments, the organoid differentiation substrate capable of forming a 3D organoid is refreshed two-daily, daily, each 2 days, each 3 days, each 4 days.

In a specific embodiment, the organoid differentiation substrate capable of forming a 3D organoid is refreshed daily.

In some embodiments, the contacting of organoid differentiation substrate on day 1 of culturing is at a one-fold, two-fold, three-fold, four-fold, five-fold, six-fold, seven-fold, eight-fold, nine-fold, ten-fold higher concentration of supplements compared to the organoid medium.

In a particular embodiment, the contacting of organoid differentiation substrate on day 1 of culturing is at a one-fold, two-fold, three-fold, four-fold, five-fold, six-fold, seven-fold, eight-fold, nine-fold, ten-fold higher concentration of supplements compared to the organoid medium.

In a following embodiment of the method of the present invention, the contacting of organoid differentiation substrate on day 1 of culturing is at a three-fold higher concentration of supplements compared to the organoid medium.

In one embodiment, on day 1 of contacting the PSCs to an organoid differentiation substrate, 10%, 15%, 20%, 25%, 30%, 35%, 40%, 45%, 50% of the organoid medium can be added with a one-fold, two-fold, three-fold, four-fold, five-fold higher concentration of supplements compared to the organoid medium.

In one embodiment, on day 1 of contacting the PSCs to an organoid differentiation substrate, 30% of the organoid medium can be added with a three-fold higher concentration of supplements compared to the organoid medium.

In some embodiments, the non-adherent culture is replated after at least 5 days, at least 6 days, at least 7 days, at least 8 days, at least 9 days, at least 10 days, at least 11 days, at least 12 days, at least 13 days, at least 14 days, at least 15 days, at least 16 days, at least 17 , at least 18 days, at least 19 days, at least 20 days, at least 21 days, at least 22 days, at least 23 days, at least 24 days, at least 25 days, at least 26 days, at least 27 days, at least 28 days, at least 29 days, at least 30 days.

In a specific embodiment of the method of the present invention, the non-adherent culture is re-plated after 7 days of culturing.

In a specific embodiment of the method of the present invention, said non-adherent culture of step e) is re-plated after 7 days of culturing to a low-binding surface.

In some embodiments, cultured PSCs are transferred to a low-binding surface, in particular an ultra-low attachment plate.

In some embodiments of the method of the present invention, the replating of the non-adherent culture is at a density of at least 5 spheres, at least 10 spheres, at least 15 spheres, at least 20 spheres, at least 25 spheres, at least 30 spheres, at least, 35 spheres, at least 40 spheres, at least 50 spheres, at least 60 spheres, at least 70 speres, 80 spheres, at least 90 spheres, at least 100 spheres per well. In a case where the density of the replating is below the lower range, the organoid cannot be properly formed. In a case where the density of the replating is above the upper range, the cells cannot be organized in a proper organoid shape.

In yet a further embodiment of the method of the present invention, the re-plating of the non-adherent culture is at a density of at least 20 spheres per well, in particular about 22 spheres per well.

In a particular embodiment of the method of the present invention, the non-adherent culture is contacted with a cell grow medium.

In some embodiments, the cell grow medium comprises one or more compounds selected from the list comprising DMEM, DMEM/F12, DMEM with high glucose or horse serum.

In a particular embodiment, the cell grow medium comprises DMEM with high glucose supplemented with horse serum.

In another particular embodiment, the concentration of horse serum in the cell grow medium is about 0.5%, 1.0%, 1.5%, 2.0%, 2.5%, 3.0%, 3.5%, 4.0%, 4.5%, 5.0%. In a particular embodiment, the cell grow medium comprises DMEM with high glucose supplemented with 2.0% horse serum.

In some embodiments, the organoid of the present invention may be cultured for 7 to 20 days until the 3-dimensional structure is formed, and the culture is preferably performed for 14 to 18 days. However, the present invention is not limited thereto. In a case where the culture time is below the lower range, the organoid may not be fully developed. In a case where the culture time exceeds the upper range, organoid cannot be kept on an orbital shaker and should be transferred onto a basement membrane matrix such as Matrigel or Geltrex.

In some embodiments, the non-adherent culture is contacted with a cell grow medium on day 7, day 8, day 9, day 10, day 11, day 12, day 13, day 14, day 15, day 16, day 17, day 18, day 19, day 20, day 21, day 22, day 23, day 24, day 25, day 26, day 27, day 28, day 29, day 30 of culture or any combination thereof.

In some embodiments, the non-adherent culture is contacted with a cell grow medium after 9 days of culturing.

In a particular embodiment of the method of the present invention, after 9 days of culturing, said non-adherent culture is contacted with a cell grow medium, in particular DMEM, more in particular DMEM with high glucose supplemented with 2.0% horse serum.

In some embodiments, the non-adherent culture is replated to a low-binding surface, in particular an ultra-low attachment plate.

In a third step of the method of the present invention, the formed neuromuscular 3D organoid is transferred to a surface comprising a basement membrane matrix to further develop a mature (step f). Organoid cultures are grown in a way that allows their expansion in three dimensions, which is achieved either by aggregating cells into 3D structures or by embedding the cultures into a 3D matrix.

In one embodiment, organoids are transferred after 1 week, 2 weeks, 3 weeks, 4 weeks, 5 weeks, 6 weeks, 7 weeks, 8 weeks, 10 weeks, in particular 2 weeks after being exposed to differentiation substrate.

In some embodiments, the organoid is maintained in culture for at least 30 days, at least 40 days, at least 50 days, at least 60 days, at least 70 days, at least 80 days, or at least 90 days.

In another embodiment of the method of the present invention, said basement membrane matrix can be Matrigel or Geltrex.

As used herein, the term "Matrigel" refers to a protein complex (product name of BD Biosciences) extracted from sarcoma cells of an EHS (Engelbreth-Holm-Swarm) mouse, and contains extracellular matrix (ECM) such as laminin, collagen, and heparin sulfate proteoglycan, and growth factors such as fibroblast growth factor (FGF), epidermal growth factor (EGF), insulin-like growth factor (IGF), transforming growth factor-beta (TGF-β), and platelet-derived growth factor (PDGF). The complex that makes up Matrigel provides a complex extracellular environment found in many tissues, which allows Matrigel to be used as a substrate for cell culture. Geltrex is similar to Matrigel (product name of ThermoFisher Scientific) and differs in the protein concentration range which is 12-18 mg/mL compared to 8-21 mg/mL for Matrigel.

In some embodiments, the volume of support matrix used for the developed organoid is at least about 10 mL, at least about 15 mL, at least about 20 mL, at least about 25 mL, at least about 30 mL, at least about 35 mL, at least about 40 mL, at least about 45 mL, or at least about 50 mL.

### EXAMPLES

The invention will now be illustrated by means of the following examples. It will be understood by those skilled in the art that various changes and modifications can be easily made without departing from the technical spirit or essential features of the present invention. Therefore, it is to be understood that the below-described examples are illustrative in all aspects and do not limit the scope of the invention in any way.

### Materials and methods:

### iPSC cell lines and maintenance

Human iPSC lines include a healthy control along with a CMT1A (PMP22 duplication) patient line (CS67iCMT, Cedars Sinai) and a CMT2A (c.281G>A, R94Q) patient line. The iPSC lines were cultured as described in Van Lent et al. 2021. Briefly, iPSCs were maintained on Matrigel coated plates (734-1440, VWR biotechnologies) in Essential 8 Flex medium and supplement (A2858501, Thermo Fisher). RevitaCell supplement (A2644501, Thermo Fisher) was used to enhance cell survival upon reviving. Medium was refreshed every alternate day and iPSCs were passaged using Versene (EDTA) (LO BE17-711E, Westburg). All the iPSC lines were tested negative for mycoplasma. All experiments performed using patient material were approved by the Committee for Medical Ethics, University of Antwerp.

### Differentiation of iPSCs into organoids

iPSC colonies where enzymatically dissociated using StemPro Accutase Cell Dissociation Reagent (A1110501, Thermo Fisher), when 80% confluency was reached. At day 0, single cells were resuspended in organoid medium consisting of STEMdiff APEL2 Medium (05270, Stemcell Technologies) and 5.32% Protein Free Hybridoma Medium II (12040077, Thermo Fisher) supplemented with 0.5 µM WNT antagonist CHIR99021 (CHIR, 4423/10, Bio-Techne), 10 ng/mL bFGF (233-FB-025, Bio-Techne), 20 µM Forskolin (sc-3562, Santa Cruz Biotechnology) and 10 µM ROCK Inhibitor (Y-27632) (688001-500, VWR biotechnologies). Single cells were plated at a density of 300.000 cells/mL on an AggreWell 800 plate (34811, Stemcell Technologies) which was pre-treated with an Anti-Adherence Rinsing Solution (07010, Stemcell Technologies). Afterwards the plate was centrifuged at 100 g for 3 min. The medium was changed every day, however the first day only 30% of the organoid medium was added with a three-fold higher concentrations of the supplements. At day 7, spheres were collected and re-plated in ultra-low binding 96-well plates (7007, Corning) at a density of 22 spheres/well. Plates were kept on an orbital shaker (KS 260 control, IKA) at 70 rpm and from day 9 onwards, half of the medium was replaced with DMEM, high glucose (41965039, Thermo Fisher) containing 2% Horse Serum (16050130, Thermo Fisher). After 2 weeks the organoids were transferred into Matrigel to further develop and mature.

### Immunohistochemical analysis

Organoids were cultured and fixed in 4% paraformaldehyde (PFA, 3105.1, Carl Roth) for 35 minutes at room temperature. Fixed cultures were permeabilized with 0.1% Triton X-100 (9002-93-1, Sigma-Aldrich), then blocked in 5% BSA (9048-46-8, Sigma-Aldrich) diluted in PBS for 1 h. The samples were incubated overnight with primary antibodies diluted in blocking buffer at 4°C. Secondary antibodies were added for 4 h, followed by a nuclear staining for 10 minutes. α-BTX-Alexa 647 and Lectin-Alexa 647 were added during the last hour of incubation. Stained cultures were imaged with a Zeiss LSM700 laser scanning confocal microscope. Antibodies for immunohistochemical analysis were α-BTX, Beta-III-Tub, BRN3A, Desmin, GFAP, Isl1, Lectin, MBP, NF-M, NF-M, S100, SOX2, T/BRA.

### Real-time qPCR

Total RNA was extracted using the Roboklon kit (E3598, GeneMATRIX) and subsequently reverse transcribed using the M-MLV Reverse Transcriptase system (28025013, Thermo Fisher). Real-time qPCR was performed using the SYBR Green dye (4368708, Thermo Fisher) and QSTUDIO6FLEX Real-Time PCR system (Thermo Fisher). Qbase+ software (Biogazelle, Zwijnaarde, Belgium) was used in the data analysis, where the relative target quantities were normalized by geometric averaging of internal reference genes (*GAPDH*, *HPRT*, *HMBS*). Primer sequences (5'-3') for internal reference genes used in this study are: GAPDH F: TGCACCACCAACTGCTTAGC & R: GGCATGGACTGTGGTCATGAG; HMBS F: GGCAATGCGGCTGCAA & R: GGGTACCCACGCGAATCAC; HPRT1 F: TGACACTGGCAAAACAATGCA & R: GGTCCTTTTCACCAGCAAGCT; MBP F: AGGATTTGGCTACGGAGGCAGA & R: GGTTTTCAGCGTCTAGCCATGG.

### Electron microscopy

For transmission electron microscopy (TEM), organoids were fixed in 2.5% glutaraldehyde containing solution. Afterwards, organoids were embedded in 2% low-melting point agarose for further processing. Next, three washes in 0.1 M sodium cacodylate-buffered (pH 7.4), containing 7.5% saccharose, were performed. Post-fixation was performed by incubating cells for 2 h with 1% OsO₄ solution. After dehydration in an ethanol gradient, samples were embedded in EM-bed812. Ultrathin sections were stained with lead citrate, and examined in a Tecnai G2 Spirit Bio TWIN microscope (Fei, Europe BV, Zaventem, Belgium) at 120 kV. Quantification of the interperiodic line distance and the g-ratio (axon diameter/fibre diameter) was performed using the Fiji distribution of ImageJ. For scanning electron microscopy (SEM), organoids were Matrigel-embedded on coverslips and afterwards fixed in 2.5% glutaraldehyde solution in 0.1 M sodium cacodylate-buffer (pH 7.4). After dehydration in an ethanol gradient (50% -70% - 90% - 95% - 100% ethanol), samples were critical point dried, mounted on a SEM-grid and sputter coated with 20 nm gold particles. Images were recorded with a JSM-IT100 SEM (Jeol, Zaventem, Belgium).

### Single cell RNA-seq and data analysis

Neuromesodermal progenitors (NMPs) and organoids were washed in PBS and dissociated using 2 mL of StemPro Accutase Cell Dissociation Reagent for 10 minutes. Afterwards, mechanically dissociating was performed using a pipette to obtain single cells, which were collected by centrifugation at 200 *g* for 4 min. Afterwards, cells were counted, and viability was assessed prior to resuspension in cryopreservation solution. Single-cell RNA-seq was performed by BGI Genomics Co., Ltd. (Shenzhen, China). Briefly, the cell suspension was prepared for 10X Genomics processing. Microfluidic inDrop encapsulation was performed followed by cell lysis, to ensure transformation into Single Cell Gel-Bead-in-Emulsions (GEMs). A reverse transcriptase (RT) reaction was carried out in the droplets, followed by demulsification to construct a cDNA library. Sequencing of the cDNA libraries was performed using the MGISEQ-2000 platform.

FASTQ files were generated and processed using Cell Ranger count pipeline (v.5.0.1), which made use of the STAR alignment software¹⁶ to align cDNA reads to the GRCh38 human reference genome. Aligned reads were then filtered for valid cell barcodes and (Unique Molecular Identifiers) UMIs to generate gene-cell matrices for downstream analysis. The Loupe Cell Browser (v.5.1.0; 10X Genomics) was used to display results.

### Statistical analysis

All experiments were at least performed in triplicates (except single cell RNA-seq), collected from independent differentiations. Statistical analysis was performed using GraphPad Prism (version 8.3.1). Normality was tested using the Shapiro-Wilk test, followed by the Mann-Whitney U test (not normally distributed) comparing two groups. Data containing three groups were analyzed with either a one-way ANOVA followed by Dunnett's multiple comparisons test (normal distribution) or a Kruskal-Wallis non-parametric test with Dunn's correction for multiple comparisons. * *P*<0.05, ** *P*<0.01, *** *P*<0.001, **** *P*<0.0001 were considered significant. Data values represented mean ± SEM, except otherwise mentioned.

### Results

### EXAMPLE 1 - Generation and characterization of iPSC-derived organoids

An organoid according to the described method was generated which captures the physiological complexity of the human PNS that cannot be reproduced by traditional 2D cultures (**Fig. 1**). iPSCs were dissociated into single cells and replated into microwells, supplemented with bFGF, CHIR and Forskolin for 7 days. At day 2, the cells became neuromesodermal precursors (NMPs), expressing both SOX2 and T/BRA (EM data not shown). These cells give rise to neuroectodermal and mesodermal cell derivatives. After 7 days, cells were transferred to ultra-low attachment plates on an orbital shaker, where the medium was gradually changed to DMEM, high glucose containing 2% Horse Serum. After 2 weeks, organoids were embedded in Matrigel to allow further maturation.

A single-cell RNA-sequencing experiment was performed at day 2 and day 20 to determine the major cell populations. The transcriptomes of 25,965 cells from day 2 cultures were analyzed using microfluidic inDrop single-cell RNA sequencing, with a mean of 14,769 reads/cell. In this early stage of differentiation, a population of ribosomal and mitochondrial marker genes were detected, playing a crucial role in early developmental processes (cluster 1, 4). The remaining transcript profiles (cluster 2, 3) contained marker genes characteristic for both neuroectodermal development as well as genes known to be progenitor cells for mesodermal development (such as *SOX2, TUBB2B, PAX6, FOXC1* and *SOX9*) (data not shown).

To investigate the cells generated during organoid development, a single-cell RNA-sequencing at Day 20 was performed. 14,040 cells were analyzed with a mean of 57,078 reads/cell. Cells were clustered into 19 independent groups (data not shown). The cluster identity was assessed using differentially expressed cluster marker genes. The neuroectodermal and mesodermal progenitor cells, detected at the early time point, further expanded into a heterogeneous organoid model. Briefly, marker genes revealed 7 broad cell populations discriminating cells involved in neural development (clusters 9, 8, 11) defined by expression of marker genes such as *SOX2*, *DRAXIN* and *RFX4,* mesenchymal cell development (clusters 6, 13) defined by the presence of *TWIST1* and *PRRX1* marker genes as well as collagen marker genes, neurons (cluster 15), glial cells (cluster 10), cells involved in lipid transport and metabolism (cluster 1, 16, 18) identified by expression of marker genes such as *APOA1, APOC2* and *FABP1,* endothelial cells (cluster 17) and erythroid cells (clusters 3, 19). Surprisingly, the presence of *SOX10, S100B* and *MPZ* (cluster 10) was detected (data not shown) which are marker genes known to be important throughout Schwann cell development. These results are in line with the cellular composition of mice peripheral nerves.

### EXAMPLE 2 - Organization and maturation of organoids

To further confirm the simultaneous development of ectodermal and mesodermal derived cell types within the organoid, we performed immunostainings. This revealed the presence of both motor (Islet1-positive) and sensory (BRN3A-positive) neurons (data not shown). Mesoderm-derived muscles were also detected, marked as desmin-positive cells. The presence of endothelial cells was visualized using tomato lectin, a widely used marker of blood vessels. As both neurons and muscle cells simultaneously develop and interact, α-bungarotoxin (aBTX) was used to reveal the formation of NMJs (EM data not shown). The presence of synaptic vesicles in the presynaptic nerve terminal (EM data not shown) was detected using electron microscopy and is indicative of functional neuromuscular units.

In addition to the neuromuscular cell types, the presence of glial fibrillary acidic protein (GFAP), an astroglial marker, and myelin basic protein (MBP), a marker for myelinated axons, was demonstrated (EM data not shown). Along with S100 protein, this confirms the presence of both glial and Schwann cells. In addition, RT-qPCR demonstrated an increase in the relative expression of *MBP* over time from iPSC colonies (Day 0) to mature organoid cultures (**Fig. 2**). To confirm these findings, transmission electron microscopy was used to demonstrate the presence of Schwann cells and stages of myelin compaction (EM data not shown). Scanning electron microscopy confirmed Schwann cells demonstrating a spindle-elongated morphology (EM data not shown).

### EXAMPLE 3 - Organoids demonstrate alterations in myelination characteristic to CMT1A patients

It was further explored whether these organoids are suitable to study CMT1A, a disease affecting myelination. First, the ultrastructural myelin architecture at Days 18-25 was investigated. During analysis of the myelin sheath thickness in the CMT1A organoid, mainly small axons (>2 µm) were detected which were subsequently found to be hypermyelinated, assessed by a decreased g-ratio (**Fig. 3A**). In addition, an increased interperiodic line distance in CMT1A (**Fig. 3B**) was shown, which was in line with previous *in vivo* data (Boutary et al. 2021). At a later developmental stage, the presence of onion bulb-like formation in the CMT1A organoid was observed (EM data not shown), which is a well-known hallmark of Schwann cell pathology in demyelinating peripheral neuropathies. These structures were different from control and CMT2A organoids, which represents axonal neuropathy.

In summary, these data show that organoid cultures for CMT1A accurately recapitulate key features of the pathology in humans, making it a suitable model for the future.

### REFERENCES

Van Lent J, Verstraelen P, Asselbergh B, et al. Induced pluripotent stem cell-derived motor neurons of CMT type 2 patients reveal progressive mitochondrial dysfunction. Brain. 2021;144(8):2471-2485.
Boutary S, Caillaud M, El Madani M, et al. Squalenoyl siRNA PMP22 nanoparticles are effective in treating mouse models of Charcot-Marie-Tooth disease type 1 A. Commun Biol. 2021;4(1):317.

## Claims

1. A method of obtaining a neuromuscular 3D organoid, the method comprising:
a) Obtaining pluripotent stem cells (PSC);
b) Culturing said PSCs in a cell culture substrate;
c) Resuspending the cells of step b) in an organoid differentiation substrate ;
d) Seeding the resuspended cells of step c) as a non-adherent culture;
e) Contacting the non-adherent culture of step d) with said organoid differentiation substrate until a neuromuscular 3D organoid is formed;
f) transferring said formed neuromuscular 3D organoid to a surface comprising a basement membrane matrix;
wherein during step d) to step f) said non-adherent culture is maintained on a shaker.

2. The method according to claim 1, wherein said organoid differentiation substrate comprises one or more reagents selected from the list comprising STEMdiff APEL2 medium, Protein Free Hybridoma Medium, NB medium, a GSK-3 Inhibitor, basic fibroblast growth factor (bFGF2), Forskolin, ROCK Inhibitor, B27 supplement, N2 supplement, L-glutamine, neurobasal medium, BSA fraction V, 2-mercaptoethanol, in particular STEMdiff APEL2 medium and Protein Free Hybridoma Medium.

3. The method according to any one of claims 1-2, wherein said organoid differentiation substrate comprises one or more reagents selected from the list comprising a GSK-3 Inhibitor, basic fibroblast growth factor (bFGF2), Forskolin.

4. The method according to any one of claims 1-3, wherein the seeding of step d) is performed on a surface pre-treated with an anti-adherence rinsing solution.

5. The method according to any one of claims 1-4, further comprising centrifuging said non-adherent culture after seeding of step d), in particular for 3 min at 100 G.

6. The method according to any one of claims 1-5, wherein said shaker is an orbital shaker, in particular an orbital shaker rotating at a speed ranging between 25 and 200 rpm, preferably 70 rpm.

7. The method according to any one of claims 1-6 further comprising dissociating said cultured PSCs into single cells prior to the resuspending of step c), in particular by a cell detachment solution, in particular accutase.

8. The method according to any one of claims 1-7, wherein the seeding of step d) is performed at a density of at least 300 000 cells/mL resuspended cells.

9. The method according to any one of claims 1-8, wherein said non-adherent culture of step e) is re-plated after 7 days of culturing to a low-binding surface.

10. The method according to claim 9, wherein the re-plating of the non-adherent culture is at a density of 22 spheres per well.

11. The method according to any one of claims 1-10, wherein after 9 days of culturing, said non-adherent culture is contacted with a cell grow medium, in particular DMEM, more in particular DMEM with high glucose supplemented with horse serum.

12. The method according to any one of claim 1-11, wherein said basement membrane matrix is Matrigel or Geltrex.

13. The method according to any one of claim 1-12, wherein said PSCs are obtained from a human subject having a neuromuscular disease, in particular a peripheral neuropathy disease, more in particular Charcot-Marie-Tooth disease.

14. A neuromuscular organoid obtained using the method of anyone of claims 1-13.

15. The neuromuscular organoid according to claim 14 for use to study neuropathic diseases, in particular CMT1A (myelination), CMT1B, CMT2a (aconopathy), CMT3-6, CMTX.
